(19) Europäisches Patentamt / European Patent Office / Office européen des brevets

(11) **EP 1 742 051 A2**

(12) **EUROPEAN PATENT APPLICATION**

(43) Date of publication:
**10.01.2007 Bulletin 2007/02**

(51) Int Cl.:
*G01N 33/48* (2006.01)   *G01N 33/53* (2006.01)

(21) Application number: **06015394.7**

(22) Date of filing: **13.02.2001**

(84) Designated Contracting States:
**AT BE CH CY DE DK ES FI FR GB GR IE IT LI LU MC NL PT SE TR**

(30) Priority: **16.02.2000   JP   2000037680**
**28.12.2000   JP   2000400574**

(62) Document number(s) of the earlier application(s) in accordance with Art. 76 EPC:
**01904384.3 / 1 256 802**

(71) Applicant: **SYSMEX CORPORATION**
**Chuo-ku**
**Kobe-shi**
**Hyogo 651-0073 (JP)**

(72) Inventors:
• **Uemura, Yahiro**
**Nishi-ku,**
**Kobe-shi,**
**Hyogo 651-2241 (JP)**

• **Hiura, Hisahide**
**Nishi-ku,**
**Kobe-shi,**
**Hyogo 651-2241 (JP)**

• **Nagamatsu, Katashi**
**Nishi-ku,**
**Kobe-shi,**
**Hyogo 651-2241 (JP)**

• **Ochiai, Koji**
**Nishi-ku,**
**Kobe-shi,**
**Hyogo 651-2241 (JP)**

(74) Representative: **Forstmeyer, Dietmar et al**
**BOETERS & LIECK**
**Oberanger 32**
**80331 München (DE)**

Remarks:
This application was filed on 24 - 07 - 2006 as a divisional application to the application mentioned under INID code 62.

(54) **Method for examination of feces occult blood**

(57) There is provided a method where a feces occult blood test with a good precision is possible regardless of the sampling precision of feces.

The above-mentioned method is achieved when the feces amount is corrected by an index being able to reduce the amount of the sampled feces to be tested in the measurement of hemoglobin and/or transferrin which are/is marker (s) for feces occult blood. Thus, together with the measurement of the feces occult blood marker, a signal obtained by the optical measurement of a suspension of the feces to be tested and/or the amount of the indigenous substances in the feces to be tested being able to be the above index and then the measured value of the feces occult blood marker is reduced using the measured value of the said index as a control whereby the above object is achieved.

EP 1 742 051 A2

**Description**

Technical Field

The present invention relates to a method for the examination of feces occult blood. More particularly, it relates to a method for the examination of feces occult blood, characterized in that, in the measurement of feces occult blood, changes in the amount of feces occult blood markers due to the difference among individuals in the sampled feces amount to be tested are corrected. The present invention further relates to a means and an apparatus used for the clinical test of feces occult blood.

Background Art

With regard to a test method for feces occult blood by bleeding of digestive tracts, there are used chemical test method and immunoassay. The chemical test method is a method where peroxidase-like activity of hemoglobin is judged by oxidative coloration of chromogen. In that case, feces is smeared on a test paper where filter paper is blotted with chromogen and an aqueous solution of hydrogen peroxide containing a coloring solution is dropped onto the smeared feces whereby a judgment is carried whether coloration takes place. On the other hand, with regard to a sample used for an immunoassay, a buffer charged in a feces-sampling container in which feces is suspended is used as a sample and hemoglobin and transferrin are measured by an immunoassay such as immunological latex agglutination method, erythrocyte agglutination method or immunochromatographic method.

As mentioned above, test of feces occult blood is carried out using hemoglobin or transferrin as a marker and it is the biggest problem in such a test to sample a certain amount of feces with a good precision. Various devices have been done for the feces-sampling container which is commercially available at present and efforts have been made for the sampling of a predetermined amount of feces. However, there are big individual difference and daily difference in the quality of feces and it cannot be said that feces is always sampled in a precise manner. As a result, deviations are resulted in the clinical data for feces occult blood and there is a problem that clinical data with a good precision are not available. The present invention is to provide a method by which a feces occult blood test with a good precision is possible regardless of the precision in the sampling of feces.

Disclosure of the Invention

The present inventors have firstly paid their attention to the fact that indigenous substances such as urobilin, urobilinogen, bilirubin, feces antigen (NFA), CEA, IgA, indole, scatole, amino acids, inorganic salts, peptides, saccharides, heparin, mucopolysaccharides, total protein, etc. are present in feces.

Then, various feces were sampled, a part thereof was dried by heating and weights before and after drying were measured whereupon the moisture content of each feces was determined. Further, the same feces was uniformly dispersed in an appropriate buffer or water and, with regard to its supernatant liquid, concentrations of various indigenous substances per weight of the feces were quantified. As a result, measured concentrations of various indigenous substances were reversely correlated to the moisture content of the sampled feces at all times and, therefore, it has been found that the measured concentration of the indigenous substance in the feces reflects the dry weight of the feces.

The present inventors have also paid their attention to the relation between the moisture content of the sampled feces and the signal obtained by an optical measurement when the sampled feces is suspended in a predetermined amount of water and found that there is a correlation between them.

Then, the present inventors have repeatedly carried out the studies and, as a result, they have found that the signal obtained by an optical measurement of the suspension of the feces to be tested or the indigenous substances in feces can be an index for correcting the amount of the feces to be tested and also found that, when measurement of the signal obtained by the optical measurement or the said substance which can be the said index is carried out together with the measurement of hemoglobin and/or transferrin which is/are to be the marker (s) for feces occult blood whereupon the measured values of hemoglobin and/or transferrin are compared and reduced, it is now possible to conduct a feces occult blood test with a good precision regardless of the precision in sampling of feces. The present invention has been now achieved as such.

Thus, the present invention comprises the followings.

1. A method for examination of feces occult blood in feces, characterized in that, an index by which the amount of the sampled feces to be tested can be corrected is used as a comparative control in the measurement of feces occult blood markers.

2. The method for examination of feces occult blood according to the above 1, wherein the index which is able to correct the amount of the feces to be tested contains any of the signals obtained by an optical measurement of at least a suspension of feces to be tested or indigenous substances in the feces to be tested.

3. The method for examination of feces occult blood according to the above 2, wherein the indigenous substance is at least one component selected from aspartate aminotransferase, alanine aminotransferase, alkaline phosphatase, lactate dehydrogenase, amylase, leucine aminopeptidase, inorganic phosphorus, magnesium, urobilin, urobilinogen, direct bilirubin, total bilirubin, feces antigen (NFA), iron, CEA, IgA, indole, scatole, amino acid, inorganic salt, peptide, saccharide, heparin, mucopolysaccharide and total protein.

4. The method for examination of feces occult blood according to the above 3, wherein the indigenous substance is at least one component from urobilin, urobilinogen, total bilirubin, feces antigen (NFA), CEA and IgA.

5. The method for examination of feces occult blood according to any of the above 1 to 4, wherein the feces occult blood marker contains at least any of hemoglobin and transferase.

6. A means for establishing the significance of a feces occult blood marker value, characterized in that, at least a means where the feces occult blood marker is measured and a means where feces occult blood marker value is reduced on the basis of the measured result of an index which is able to correct the amount of the sampled feces to be tested are combined.

7. The means for establishing the significance of a feces occult blood marker value according to the above 6, wherein the index which is able to correct the amount of the feces to be tested contains any of the signals obtained by an optical measurement of at least a suspension of feces to be tested or indigenous substances in the feces to be tested.

8. An apparatus which carries at least a means for establishing the significance of a feces occult blood marker value mentioned in the above 6 or 7.

Brief Description of the Drawings

Fig. 1 comprises drawings which show the correlation between moisture content in feces and absorbance. Fig. 1-1 is a drawing which shows the absorption at the wavelength of 400 nm and Fig. 1-2 is a drawing which shows the absorption at the wavelength of 550 nm.

Fig. 2 comprises drawings which show the deviation of concentrations of hemoglobin before and after the correction of the feces amount.

Fig. 3 is a drawing which explains an apparatus for immunochromatographic test. In the drawing, 1 is a sample dropping part; 2 is a site for holding a colored latex mixture labeled with anti-CEA antibody or anti-hemoglobin antibody; 3 is a nitrocellulose membrane; 4 is a zone where anti-hemoglobin antibody is fixed; 5 is a zone where anti-CEA antibody is fixed; and 6 is an absorption pad.

Best Mode for Carrying Out the Invention

In the present invention, a feces occult blood marker is a marker for testing the small amount of blood in the feces and its examples are hemoglobin and transferrin. It has been recognized by the persons skilled in the art that, in the test of feces occult blood, they can be usually used as markers for judging whether the occult blood is positive or negative.

There is no particular limitation for the measuring method of hemoglobin or transferrin and, usually, the measurement is carried out immunologically. The publicly known methods are a method where a test substance is made to react with latex particles with which an antibody specific to the substance to be tested is adsorbed and the resulting agglutination of the particles is measured either by naked eye or by an optical means; a method where metal colloid particles are used instead of the latex particles and the agglutination reaction is optically measured; and a method where measurement is carried out by means of an immunochromatography using an antibody labeled with colored latex particles or metal colloid particles.

The index which is able to correct the amount of the feces to be tested in the present invention is an index which is able to reflect the dry weight of the feces and, by the said index, amount of the sampled feces can be corrected. For example, various indigenous substances are present in the feces and measured value for those indigenous substances reflects the dry weight of the sampled feces whereby the amount of the feces can be corrected.

Examples of the indigenous substance in the feces according to the present invention are aspartate aminotransferase, alanine aminotransferase, alkaline phosphatase, lactate dehydrogenase, amylase, leucine aminopeptidase, inorganic phosphorus, magnesium, urobilin, urobilinogen, direct bilirubin, total bilirubin, feces antigen (NFA), iron, CEA, IgA, indole, scatole, amino acid, inorganic salt, peptide, saccharide, heparin, mucopolysaccharide and total protein. With regard to any other substance, its measured value can be used as an index for calculating the sampled feces amount into a predetermined feces amount so far as it is a substance which is indigenously present in the feces in a certain amount. Each of those indigenous substances may be used solely or two or more thereof may be used jointly. When two or more are used jointly, it is possible to carry out the test of the feces occult blood in a more precise manner.

It is preferred that the indigenous substance can be measured in a simpler manner and can be measured in the same system as the measuring system for hemoglobin and/or transferrin which are feces occult blood marker (s) (hereinafter, just referred to as "feces occult blood marker") . Examples of the indigenous substance to which such a measuring system is applicable are urobilin, urobilinogen, bilirubin, feces antigen (NFA), CEA and IgA.

In measuring the indigenous substances, it is possible to measure by applying a known measuring method which is intrinsic to the substance to be measured.

It is also possible that measurement of the feces occult blood marker and measurement of the indigenous substance are measured using the same instrument or apparatus. For example, when hemoglobin and/or transferrin are/is measured utilizing a latex agglutination reaction by an optical means, an optical measurement is selected for the measurement of the indigenous substance as well.

On the other hand, it is also possible to compare the measured values obtained by an entirely different principle from the measuring principle for feces occult blood such as by a combination of a latex agglutination method and a conductivity or ion-selective electrode method.

As an index which is able to correct the amount of the feces to be tested, a signal obtained by an optical measurement of a feces suspension or a mixture of the feces suspension and a reagent for testing the feces occult blood in feces can be utilized as a signal as well. Examples of the signal obtained by an optical measurement according to the present invention are absorbance, transmissivity, reflection, scattering and fluorescence and a preferred one is absorbance. For example, absorbance can be measured at various wavelengths and is obtained by measuring the absorbance preferably at the wavelength of 200~800 nm or, more preferably, 340~600 nm. It is also possible to obtain the absorbance by measuring two or more wavelengths and it is further possible that the said signal is obtained by calculation of those measured values.

When the indigenous substances in feces and/or at least one of the substances indigenously present in feces are/is measured together with the measurement of the feces occult blood marker or a signal obtained by an optical measurement of the feces suspension is measured and such a value or plural values is/are used as an index which is able to correct the amount of the feces to be tested for reducing the feces occult blood marker values, it is now possible to carry out a feces occult blood test with a good precision regardless of the precision of sampling of the feces.

According to the present invention, it is possible to establish the significance of a feces occult blood marker value by such a manner that at least a means where the feces occult blood marker is measured and a means where feces occult blood marker value per feces amount is reduced from the measured value of the feces occult blood marker on the basis of the measured result of an index which is able to reduce the amount of the sampled feces to be tested are combined.

With regard to a means for calculating the reduced value of the feces occult blood marker from the measured value of the indigenous substance, the reduction may be carried out with a prerequisite that the indigenous substance is present in the feces in a predetermined amount and is not particularly limited. In the present invention, there is provided a method where the existing ratio of the feces occult blood marker to the indigenous substance is calculated so that the feces occult blood in the feces is tested.

With regard to a method for obtaining a reduced value of the feces occult blood marker from a signal obtained by an optical measurement of the feces suspension, the reduction may be carried out with a prerequisite that a predetermined amount of the above-mentioned signal is resulted in a predetermined feces and there is no particular limitation therefor. The present invention also provides a method where the existing ratio of the feces occult blood marker to the above-mentioned signal is calculated so that the feces occult blood in the feces is tested.

The present invention further provides an apparatus which carries at least the above-mentioned means for measuring the feces occult blood marker, a means for measuring the index which is cable to correct the amount of the feces to be tested and a means for reducing the feces occult blood marker amount on the basis of the said index. As one of the constitutions of the apparatus of the present invention, an automatic analysis apparatus which is generally used for clinical tests may be utilized and, for example, there is provided an apparatus where a means for reducing the feces occult blood marker amount on the basis of the measuring result of the indigenous substance in the feces is carried in the said automatic analysis apparatus so that the process from the measurement until obtaining the reduced amount is automatically carried out.

The present invention will be more specifically illustrated hereunder using CEA as an example of the indigenous substance.

Thus, latex particles where an antibody to CEA is adsorbed are made to react with a sample and subjected to an optical measurement using an automatic analysis apparatus by which a latex agglutination reaction is commonly used so that concentration of the CEA is measured. In the same manner, concentration of hemoglobin is measured by means of an optical measurement using the same automatic analysis apparatus and using latex with which an anti-hemoglobin antibody is adsorbed. In the case of transferrin, it is also possible to measure by the same manner using latex with which an anti-transferrin antibody is adsorbed.

It is further possible to design so as to be able to measure hemoglobin, transferrin and CEA in the same apparatus using a principle of immunochromatography. As a more specific embodiment of the immunochromatography, an explanation will be made taking the case of hemoglobin and CEA as an example.

Anti-hemoglobin antibody is adsorbed with colored latex particles, metal colloid such as gold colloid, inorganic non-metallic colloid, etc. to prepare the particles labeled with anti-hemoglobin antibody. As same as in the case of hemoglobin, the particles labeled with anti-CEA antibody are also prepared by means of adsorption of anti-CEA antibody with the same or different type of particles.

The labeled particles prepared separately are mixed in an appropriate quantity ratio and carried using, if necessary, by way of an appropriate porous support. At that time, a carrier protein such as bovine serum albumin, gelatin or casein may be present together. Further, the labeled particles may be used in a dried state together with a support. With regard to a support for immunochromatography, there may be used glass filter paper or membrane having an appropriate pore size.

Antibodies (anti-hemoglobin antibody and anti-CEA antibody in the present example) trapping the labeled particles are fixed in lines at appropriate intermediate positions of the support for immunochromatography. The support carrying the labeled particles is set at one end of the support for immunochromatography and a test body sample solution is supplied to the said support for labeled particles either directly or indirectly whereupon hemoglobin and CEA in the test body immunologically react with the labeled particles. The reacted labeled particles chromatographically move on the support for immunochromatography and react with the trapped antibodies. Intensity of the reaction lines which appear after the reaction is optically measured.

That is carried out by visible light in case colored latex particles are used as labeled particles, by fluoresce in case fluorescent particles are used as those and by emission measurement in the case of emission labeling. With regard to the labeled particles used therefor, there is no particular limitation so far as it is able to detect the signal which is an object. It is also possible to use two or more kinds of particles having different measuring wavelengths.

As a method for the measurement of urobilin, CEA, IgA, peptide, etc. which are other indigenous substances, their $OD_{280nm}$ was measured. In the case of an inorganic salt, total inorganic salt concentration was measured as an amount corresponding to NaCl using a conductivity meter using an NaCl solution having a known concentration as a control.

Examples

The present invention will now be further illustrated by way of the following Examples although the present invention is not limited to the Examples.

(Example 1)

Each of the fresh feces of 40 cases (28 males and 12 females) was well mixed up to homogenize and 1~4 g thereof was/were precisely weighed in a container and used as a sample for each of the following experiments.

(Experimental Example 1-1)

Samples of the 40 cases were frozen at -80°C for 2 hours, dried *in vacuo* in a desiccator using a vacuum pump and the weight after drying was precisely weighed. Difference in the weights before and after drying was divided by the weight before drying to calculate the moisture content in each feces. The result is shown in Table 1.

Table 1

| | | Moisture Content (%) | | | | | | | | Total | Ratio (%) |
|---|---|---|---|---|---|---|---|---|---|---|---|
| | | 50-55 | 56-60 | 61-65 | 66-70 | 71-75 | 76-80 | 81-85 | 86-90 | | |
| Age | 0-10 | | | | | | 2 | | | 2 | 5 |
| | 11-20 | | | | 1 | 1 | | | | 2 | 5 |
| | 21-30 | | | 1 | | 1 | 2 | 1 | 1 | 6 | 15 |
| | 31-40 | | | | 2 | | 3 | 4 | | 9 | 23 |
| | 41-50 | 1 | | 2 | 3 | 2 | 1 | 2 | 1 | 12 | 30 |
| | 51- | | | | 1 | | 1 | 4 | 1 | 7 | 18 |
| | Unknown | | | | | | | 1 | 1 | 2 | 5 |
| | Total | 1 | | 3 | 7 | 4 | 9 | 12 | 4 | 40 | 100 |
| | Ratio (%) | 3 | 0 | 8 | 18 | 10 | 23 | 30 | 10 | 100 | |

(Experimental Example 1-2)

Water was added so as to make the concentration of each feces of the 40 cases 0.1 g/ml and the mixture was allowed to stand for a whole day and night at 2~8°C, suspended homogeneously and centrifuged at 2,500 rpm for 20 minutes to recover the supernatant liquid. The supernatant liquid was filtered using a membrane filter of 5 $\mu$m and 1.2 $\mu$m to give feces extracts. For each of the feces extracts, various components and absorbance at each wavelength were measured and an index being able to be used for correction of feces amount was investigated.

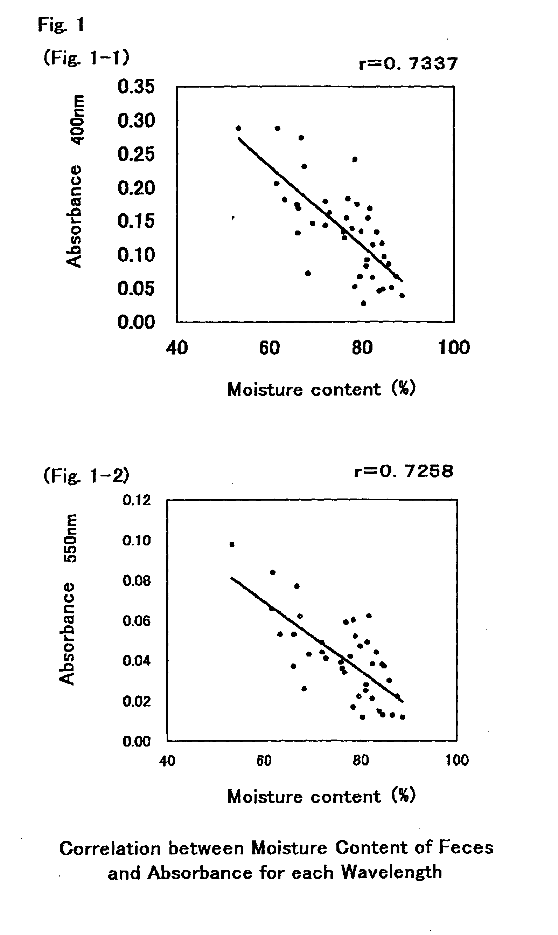

As a result, it was found that the measured values of aspartate aminotransferase, alanine aminotransferase, alkaline phosphatase, lactate dehydrogenase, amylase, leucine aminopeptidase, inorganic phosphorus, magnesium, iron, IgA, direct bilirubin, total bilirubin and absorbance reversely correlated to the moisture content and that the said measured value is useful as an index for reducing the feces amount to be tested to a predetermined feces amount. Among those, total bilirubin and absorbance showed correlation coefficient of 0.64~0.76 to the moisture content and found to be more preferred. As an example thereof, the correlation diagram of the absorbance at the wavelength of 400 nm and 550 nm to the moisture content is shown in Fig. 1.

(Experimental Example 1-3)

Feces from 10 cases was used and the correlation formula of absorbance of the feces to be tested to the feces concentration was determined. Preparation was carried out so as to make each feces concentration 1, 5, 10 and 17.5 mg/dl, then each feces solution (100 $\mu$l) was mixed with 1 ml of 20 mM phosphate buffer (pH 6.8) and the absorbance at the wavelength of 450 nm was measured.

As a result, the correlation formula of the feces concentration (C) to the absorption (A) was found to be

$$A = 0.0188C + 0.0022$$

whereby the absorbance and the feces concentration were correlated by such a formula of the first degree. It was therefore found that, when the absorbance was measured, correction of the feces was possible.

(Example 2)

Three cases where feces occult blood was positive were used and each feces was sampled for ten times using a commercially available feces sampling container (manufactured by International Reagents Corporation). Hemoglobin concentration of each sample was measured using a feces occult blood measuring kit by a latex turbidimetry (manufactured by Shima Kenkyusho) according to the directions for use attached thereto.

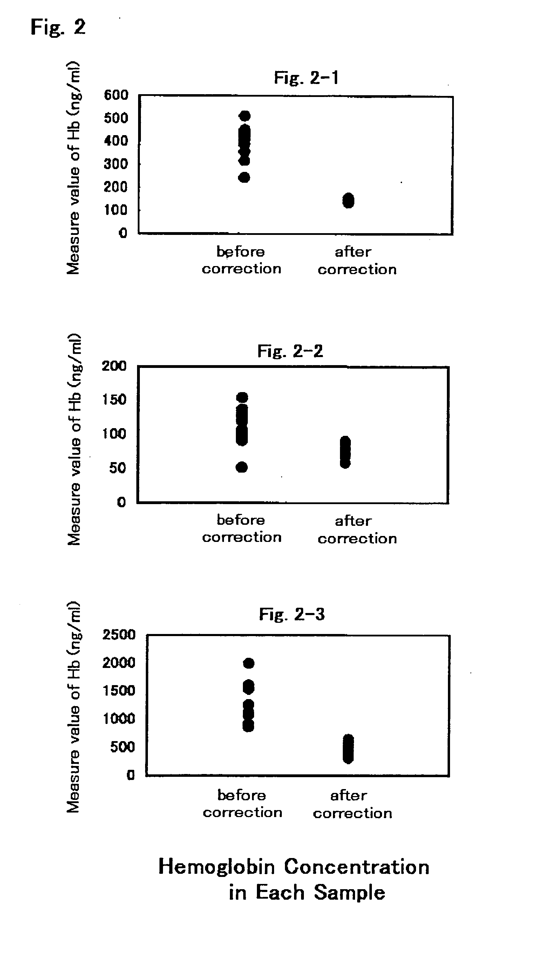

The results were 243~512, 52~154 and 857~1997 ng/ml for each. On the other hand, when the hemoglobin concentration was corrected using the correlation formula determined in Experimental Examples 1~3 on the basis of the absorbance at the wavelength of 450 nm, they were 134~155, 59~90 and 314~641 ng/ml, respectively and convergence of error by sampling of feces was noted. The result is shown in Fig. 2.

(Example 3)

(Experimental Example 3-1)

Feces of the patient where feces occult blood was positive was homogeneously mixed and feces was sampled from 10 areas using a commercially available feces sampling container which was designed in such a manner that 10 mg of sampled feces were diluted with 2 ml of the buffer. Concentration of hemoglobin (Hb) in each of the sampled samples was measured using a latex reagent with which two kinds of anti-human hemoglobin antibody (monoclonal antibody) were adsorbed. At the same time, CEA was also measured using the commercially available EIA kit for the measurement of CEA (manufactured by International Reagents Corporation).

On the basis of the measured values, there were measured mean value, maximum value/minimum value and variation coefficient for Hb concentration itself. Ratio of the measured value for each Hb to the value for CEA was calculated, then each of mean value, maximum value/minimum value and variation coefficient was calculated and the result is shown in Table 2. Incidentally, Hb amount (ng/ml) and CEA amount ($\mu$g/ml) of the patients where feces occult blood was negative were 40~3 and 2.25~0.48, respectively and a comparative value thereof was 10~30.

Table 2

| Measured Values of Hb and CEA | | | |
|---|---|---|---|
| **Feces Sampling No.** | Hb (ng/ml) | CEA ($\mu$g/ml) | (Hb/CEA)$\times$1000 |
| 1 | 583 | 2.35 | 248 |
| 2 | 126 | 0.63 | 201 |
| 3 | 252 | 1.09 | 231 |
| 4 | 328 | 1.29 | 255 |
| 5 | 478 | 2.18 | 219 |
| 6 | 139 | 0.50 | 276 |
| 7 | 184 | 0.84 | 218 |
| 8 | 265 | 1.17 | 226 |
| 9 | 347 | 1.43 | 243 |
| 10 | 261 | 1.03 | 254 |
| Mean value | 296 | 1.25 | 237 |
| **Maximum/Minimum** | 583/126 | 2.35/0.50 | 276/201 |
| **Variation coefficient (%)** | 49 | 48.43 | 9.4 |

The result was that, although the same feces was measured in the case of measurement of sole hemoglobin, variation coefficient was about 50% showing a very big variation while, when the ratio of hemoglobin to CEA was calculated using CEA which was a indigenous substance as an index, the variation coefficient was 10% or less whereby the measurement precision was significantly improved.

(Experimental Example 3-2)

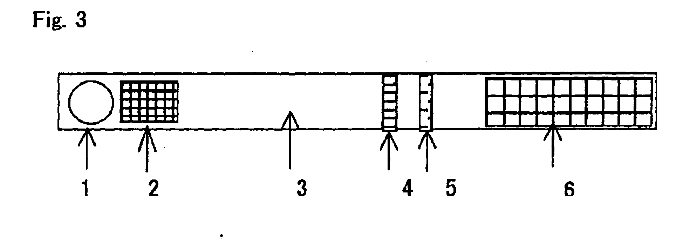

An apparatus for immunochromatographic test was prepared as shown in Fig. 3 in which there were formed a zone where anti-human hemoglobin antibody was fixed and a zone where anti-CEA antibody was fixed on a nitrocellulose membrane filter (pore size: 0.8 $\mu$m) for immunochromatography and there was further formed a site holding a mixture of colored latex (red) labeled with anti-hemoglobin antibody and colored latex (blue) labeled with anti-CEA antibody.

A suspension (about 200 $\mu$l) of the feces sampled in Experimental Example 3-1 was dropped into the said apparatus for the test, reaction was carried out for 10 minutes, optical intensities of the hemoglobin band and the CEA band were measured by measuring the light of reflection and their intensity ratio was determined. The result is shown in Table 3.

The result was that the variation coefficient was 52.8% in the case of Hb only while, when its ratio to CEA was determined, the variation coefficient became 11.4% whereby the precision in the measurement was greatly improved.

Table 3

| Result of Measurement of Hb and CEA by Immunochromatography | | | |
|---|---|---|---|
| **Feces Sampling No.** | **Reflection Intensity of Hb** | **Reflection Intensity of CEA** | **Hb/CEA** |
| 1 | 620 | 568 | 1.09 |
| 2 | 135 | 155 | 0.87 |
| 3 | 263 | 258 | 1.02 |
| 4 | 313 | 368 | 0.85 |
| 5 | 453 | 579 | 0.78 |
| 6 | 128 | 163 | 0.79 |
| 7 | 157 | 175 | 0.90 |
| 8 | 278 | 346 | 0.80 |
| 9 | 375 | 425 | 0.88 |
| 10 | 218 | 264 | 0.83 |
| Mean value | 294 | 330 | 0.89 |
| **Maximum/Minimum** | **620/128** | **579/155** | **1.09/0.79** |
| **Variation coefficient (%)** | 52.8 | 47.5 | 11. 4 |

Industrial Applicability

As a result of the above, when feces amount is corrected by an index being able to correct the sampled feces amount to be tested in the measurement of feces occult blood marker, a feces occult blood test with a good precision is now possible regardless of the sampling precision of the feces.

Preferred embodiments :

1. A method for examination of feces occult blood in feces, characterized in that, an index by which the amount of the sampled feces to be tested can be corrected is used as a comparative control in the measurement of feces occult blood markers.

2. The method for examination of feces occult blood according to claim 1, wherein the index which is able to correct the amount of the feces to be tested contains any of the signals obtained by an optical measurement of at least a suspension of feces to be tested or indigenous substances in the feces to be tested.

3. The method for examination of feces occult blood according to claim 2, wherein the indigenous substance is at least one component selected from aspartate aminotransferase, alanine aminotransferase, alkaline phosphatase, lactate dehydrogenase, amylase, leucine aminopeptidase, inorganic phosphorus, magnesium, urobilin, urobilinogen, direct bilirubin, total bilirubin, feces antigen (NFA), iron, CEA, IgA, indole, scatole, amino acid, inorganic salt, peptide, saccharide, heparin, mucopolysaccharide and total protein.

4. The method for examination of feces occult blood according to claim 3, wherein the indigenous substance is at least one component from urobilin, urobilinogen, total bilirubin, feces antigen (NFA), CEA and IgA.

5. The method for examination of feces occult blood according to any of claims 1 to 4, wherein the feces occult blood marker contains at least any of hemoglobin and transferase.

6. A means for establishing the significance of a feces occult blood marker value, characterized in that, at least a means where the feces occult blood marker is measured and a means where feces occult blood marker value is reduced on the basis of the measured result of an index which is able to correct the amount of the sampled feces to be tested are combined.

7. The means for establishing the significance of a feces occult blood marker value according to claim 6, wherein the index which is able to correct the amount of the feces to be tested contains any of the signals obtained by an optical measurement of at least a suspension of feces to be tested or indigenous substances in the feces to be tested.

8. An apparatus which carries at least a means for establishing the significance of a feces occult blood marker value mentioned in the claim 6 or 7.

**Claims**

1. A method for examination of feces occult blood in feces, **characterized in that**, a suspension of feces to be tested is optically measured as a comparative control in a measurement of feces occult blood markers, the result is used to establish and index, and the index is able to reflect the amount of sample feces being tested.

2. The method for examination of feces occult blood according to claim 1, wherein the index contains any of the signals obtained by an optical measurement of at least the suspension of feces to be tested.

3. The method for examination of feces occult blood according to claim 1 or 2, wherein the feces occult blood marker contains at least any of hemoglobin and transferase.

4. The method for examination of feces occult blood according to any of claims 1 to 3, wherein the result comprises an absorbance of the suspension.

Fig. 1

(Fig. 1-1)

r=0. 7337

(Fig. 1-2)

r=0. 7258

Correlation between Moisture Content of Feces
and Absorbance for each Wavelength

## Fig. 2

### Fig. 2-1

Measure value of Hb (ng/ml)

before correction — after correction

### Fig. 2-2

Measure value of Hb (ng/ml)

before correction — after correction

### Fig. 2-3

Measure value of Hb (ng/ml)

before correction — after correction

## Hemoglobin Concentration in Each Sample

Fig. 3